# EUROPEAN PATENT SPECIFICATION

(11) **EP 2 936 364 B1**
(45) Date of publication and mention of the grant of the patent: **25.09.2019**
(21) Application number: 13834392.6
(22) Date of filing: 12.12.2013
(51) Int. Cl.: A61B 5/02, A61B 6/00

(54) **METHOD AND APPARATUS FOR SIMULATING BLOOD FLOW UNDER PATIENT-SPECIFIC BOUNDARY CONDITIONS DERIVED FROM AN ESTIMATED CARDIAC EJECTION OUTPUT**
VERFAHREN UND VORRICHTUNG ZUR SIMULATION EINES BLUTFLUSSES AUF GRUNDLAGE EINER GESCHÄTZTEN HERZAUSWURFLEISTUNG UNTER PATIENTENSPEZIFISCHEN GRENZBEDINGUNGEN
PROCÉDÉ ET APPAREIL DE SIMULATION DE DÉBIT SANGUIN DANS DES CONDITIONS LIMITES SPÉCIFIQUES AU PATIENT OBTENUES À PARTIR D'UNE SORTIE D'ÉJECTION CARDIAQUE ESTIMÉE

(30) Priority: 18.12.2012 US 201261738562 P
(43) Date of publication of application: 28.10.2015
(73) Proprietor: Koninklijke Philips N.V., 5656 AE Eindhoven (NL); Philips GmbH, 20099 Hamburg (DE)
(72) Inventor: PETERS, Jochen, NL-5656 AE Eindhoven (NL); WEESE, Juergen, NL-5656 AE Eindhoven (NL); SCHMITT, Holger, 5656 AE Eindhoven (DE)
(74) Representative: Versteeg, Dennis John
(86) International application number: PCT/IB2013/060841
(87) International publication number: WO 2014/097063

(56) References cited:
- US-A1- 2007 014 452
- US-A1- 2008 294 057
- US-A1- 2012 041 318
- Moloy Kumar Banerjee ET AL: "Effect of Pulsatile Flow Waveform and Womersley Number on the Flow in Stenosed Arterial Geometry", ISRN Biomathematics, 1 November 2012 (2012-11-01), XP055446388, Retrieved from the Internet: URL:https://www.hindawi.com/journals/isrn/ 2012/853056/ [retrieved on 2018-01-31]

## Description

### FIELD OF THE INVENTION

The invention relates to the field of simulation of blood flow through a target cardiovascular structure, such as - but not limited to - a patient-specific geometry of the left ventricular outflow tract, the aortic root including the aortic valve (AV) and the ascending aorta, based on information acquired through medical imaging techniques.

### BACKGROUND OF THE INVENTION

Degenerative aortic valve stenosis (AS) is the second most common cardiovascular disease with an incidence of 2-7% in the Western European and North American populations aged beyond 65 years, as described in G.M. Feuchtner, W. Dichtl, et al. "Multislice Computed Tomography for Detection of Patients With Aortic Valve Stenosis and Quantification of Severity", Journal of the American College of Cardiology 2006, 47 (7), 1410-1417.

Management of patients with degenerative AS depends on the severity of the disease. Assessment of the severity of the stenosis of an aortic valve (AV) can involve different imaging modalities. Current assessment of the severity is mostly based on ultrasound and Doppler measurements or on geometric measurements derived from magnetic resonance imaging (MRI) or computed tomography (CT) images of the AV area.

For ca. 60-70% of the patients, ultrasound can be used to image the valve and to measure blood velocities via Doppler measurements. For a stenosed valve, due to the reduced effective opening area, the blood has to flow at higher velocities and the results of the Doppler measurements can be taken as indicator of aortic stenosis (AS).

Using electrocardiography (ECG) gating, CT and MRI allows to reconstruct or acquire images from a selected narrow cardiac phase interval and gives access to images that show the valve in the relatively short open state. G.M. Feuchtner, W. Dichtl, et al. "Multislice Computed Tomography for Detection of Patients With Aortic Valve Stenosis and Quantification of Severity", Journal of the American College of Cardiology 2006, 47 (7), 1410-1417 and Y. Westermann, A. Geigenmüller, et al. "Planimetry of the aortic valve orifice area: Comparison of multi-slice spiral CT and MRI", European Journal of Radiology 2011, 77, 426-435, suggest using images of the open valve to measure the valve opening using a few selected angulated cut planes and delineating the apparent valve orifice. The measured area of this orifice is then used to assess the degree of stenosis. This technique is called AV area planimetry.

However, for planimetry measurements of the AV area from CT or MRI, only a two-dimensional (2D) cut is analyzed. Whether the valve leaflets meet at commissure lines in some other region downstream is not analyzed. The impact on the three-dimensional (3D) blood flow can therefore not be fully assessed by such 2D measurements. The relation between such measured areas and the physiological impact of a stenosed valve, such as increased pressure gradients, is thus unclear.

US 2012/0041318 A1 discloses a system for determining cardiovascular information for a patient. The system may include at least one computer system configured to receive patient-specific data regarding a geometry of the patient's heart, and create a three-dimensional model representing at least a portion of the patient's heart based on the patient-specific data. The at least one computer system may be further configured to create a physics-based model relating to a blood flow characteristic of the patient's heart and determine a fractional flow reserve within the patient's heart based on the three-dimensional model and the physics-based model.

US 2007/0014452 A1 discloses a computerized method of facilitating cardiac intervention. The method may include inputting patient data and creating a computerized interactive model of a heart based on the patient data. The model may include features. The model may simulate at least one proposed cardiac intervention by adding or deleting features to the model, and determining the effects of the proposed cardiac simulation upon the entire model. Simulations may be repeated to allow the user to determine an optimal cardiac intervention. A template and/or patient specific instrument may be created from the model to use as a guide during the cardiac intervention.

US 2008/0294057 A1 discloses methods and systems for estimating cardiac ejection fraction, cardiac contractility, and ventricular end-diastolic volume on a beat-by-beat basis include observing arterial blood pressure waveforms to determine ventricular compliances for a pressure-volume loop in the ventricle. Uncalibrated or calibrated cardiac ejection fraction may be calculated from estimates of stroke volume and the ventricular compliances. Cardiac contractility may be calculated from estimates of a ventricular compliance. Uncalibrated or calibrated ventricular end-diastolic volume may also be calculated from estimates of stroke volume and the ventricular compliances. A set of calibration parameters for calibrating cardiac ejection fraction or ventricular end-diastolic volume may be estimated in a least-squares manner.

### SUMMARY OF THE INVENTION

It is an object of the invention to derive objective values for physiological parameters of a patient under consideration from patient-specific boundary conditions.

This object is achieved by an apparatus as claimed in claim 1, a method as claimed in claim 4, and a computer program product as claimed in claim 13.

Accordingly, a volumetric mesh of the cardiovascular structure including the left ventricle outflow tract, the aortic root including the aortic valve (AV), plus ascending aorta is generated based on a partitioned digital image of the cardiovascular structure, and a cardiac ejection output per heart stroke is estimated in terms of its amount or temporal behavior from volumes of the left ventricle of the patient in different filling states at two or more different points in time. At least one patient-specific boundary condition (which may include a time-dependent boundary condition) of the cardiovascular structure is then derived from the cardiac ejection output per heart stroke and the simulated blood flow is obtained by simulating a blood flow through the volumetric mesh under consideration of the patient-specific boundary conditions.

Thus, blood flow through a patient-specific geometry of a target cardiovascular structure under patient-specific boundary conditions is derived from the cardiac ejection output per heart stroke. The result of the simulation yields objective values for physiologically relevant parameters such as one or more of pressure drop, average blood residence time, flow rate, wall sheer stress and blood swirl in said cardiovascular structure. According to a first aspect, a modeling circuit may be provided for generating the volumetric mesh of the cardiovascular structure based on a partitioned segmented digital image of the cardiovascular structure. Thereby, the volumetric mesh can be directly generated and does not need to be derived or loaded from a remote device or network.

According to a second aspect which can be combined with the above first aspect, the digital image may be a CT image or an MRI image or an ultrasonic image. Thus, the proposed solution can be used for a wide range of medical imaging systems.

According to a third aspect which can be combined with the above first or second aspect, the digital image may be partitioned by using a model-based segmentation to obtain a surface mesh of the target cardiovascular structure. Thereby, the volumetric mesh can be readily obtained by converting or transforming the surface mesh into the volumetric mesh.

According to a fourth aspect which can be combined with any one of the above first to third aspects, the simulation may be done by computational fluid dynamics (CFD) or fluid-solid interaction (FSI) simulation. This facilitates automation of the process of creating computer models.

According to a fifth aspect which can be combined with any one of the above first to fourth aspects, the cardiac ejection output per heart stroke maybe estimated based on electrocardiography (ECG) gated digital images. This measure ensures proper timing of image generation.

According to a sixth aspect which can be combined with any one of the above first to fifth aspects, the cardiac ejection output per heart stroke maybe estimated based on digital images of the ventricle in a maximum filling state and a minimum filling state. This provides a straight forward solution based on the two images. In a specific example, the cardiac ejection output per heart stroke may be estimated based on volumes of the left ventricle of the patient at an end of systole and at an end of a diastole.

According to a seventh aspect which can be combined with any one of the above first to sixth aspects, the estimated cardiac ejection output per heart stroke may be used to define a blood flow from a cardiac chamber to the cardiovascular structure. According to a specific example of the fifth aspect, the at least one patient-specific boundary condition may be derived by estimating a flow profile across the ventricular outflow tract and its temporal behavior. Hence, (flow) boundary conditions (e.g. to estimate the pressure drop across the target cardiovascular structure via (CFD or FSI) simulation) can be determined based on the ventricular ejection fraction by image analysis. Thereby, a complete heart simulation is no longer required, which is extremely time consuming, extremely complicated and cannot always be done with available clinical data. The flow profile may be estimated by defining a quadratic profile or a velocity profile of a pulsatile flow.

It shall be understood that the apparatus of claim 1, the method of claim 5 and the computer program product of claim 15 have similar and/or identical preferred embodiments, in particular, as defined in the dependent claims.

It shall be understood that a preferred embodiment of the invention can also be any combination of the dependent claims with the respective independent claim.

These and other aspects of the invention will be apparent from and elucidated with reference to the embodiments described hereinafter.

### BRIEF DESCRIPTION OF THE DRAWINGS

In the drawings:
Fig. 1 shows a schematic block diagram of generation and use of patient-specific boundary conditions for simulating blood flow according to an embodiment of the present invention;
Figs. 2a-f show schematic reformatted views of segmented valves for the cases of an open, calcified medium open and closed valve, respectively;
Fig. 3 shows a diagram with volume curves of heart chambers extracted by model-based segmentation; and
Fig. 4 shows a schematic exemplary visualization of a simulation of blood flow through an aortic valve.

### DETAILED DESCRIPTION OF EMBODIMENTS

Embodiments are now described based on a simulation of the blood flow through a patient-specific geometry of a left ventricle (LV) outflow tract plus ascending aorta (as an example for a blood cavity or cardiovascular structure close to the heart) under patient-specific boundary conditions derived from the cardiac ejection output per heart stroke, which blood volume can be calculated from (at least) two images of the LV in maximum and minimum filling state (e.g., end of diastole, end of systole). The geometry of the LV outflow tract, the aortic root including the AV, plus ascending aorta and the ventricle volumes can automatically be obtained by model-based segmentation.

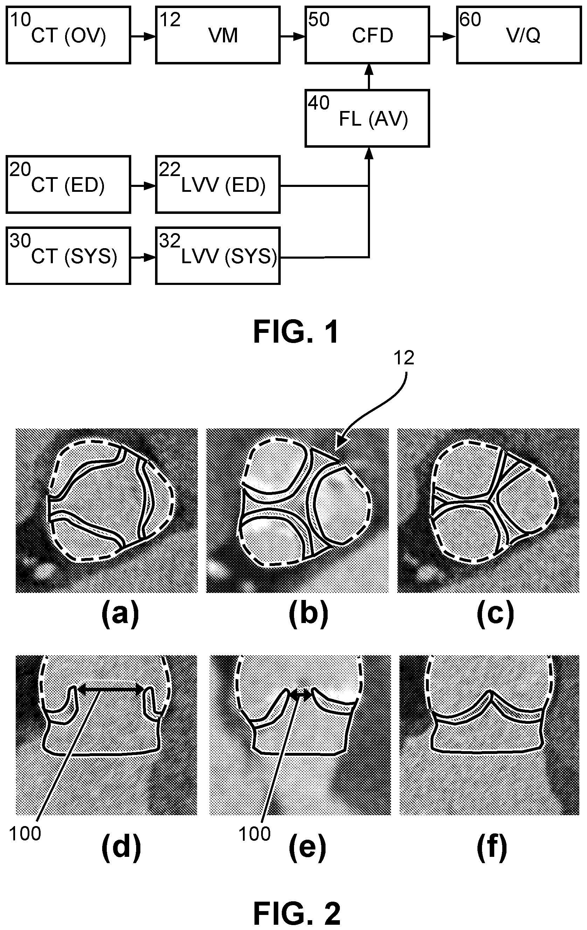

Fig. 1 shows a schematic block diagram illustrating the generation and use of patient-specific boundary conditions for simulating the flow through the aortic valve. The blocks of Fig. 1 can be regarded as hardware circuits adapted to perform the respective function or as steps of a corresponding method or process which may be implemented as software programs comprising code means for producing the related function when run on a computer or processor system.

First, in a segmentation step or circuit (CT (OV)) 10, the LV outflow tract, the aortic root including the AV, plus ascending aorta is segmented for an open valve (OV) state in a CT image to obtain a surface mesh of the complete blood cavity. This can be done using model-based segmentation as described for example in O. Ecabert, et al. "Segmentation of the heart and great vessels in CT images using a model-based adaptation framework" Medical Image Analysis 2011, 15(6), 863-876. In general, segmentation is the process of partitioning a digital image into multiple segments (i.e. sets of pixels). In the segmentation, a voxel is allocated to a specific structure, e.g., by adding a label or flag or color or outline or the like. This may be achieved by typical image segmentation methods such as thresholding, edge detection, region growing or the like. Then, in a modeling step or circuit (VM) 12, from the surface mesh obtained from the segmentation step or circuit 10, a volumetric mesh for computational fluid dynamics (CFD) or fluid-solid interaction (FSI) or other types of simulations is generated using known meshing tools. Suitable meshing tools (e.g. TetGen or NetGen) may involve techniques to generate different tetrahedral meshes from three-dimensional point sets or domains with piecewise linear boundaries.

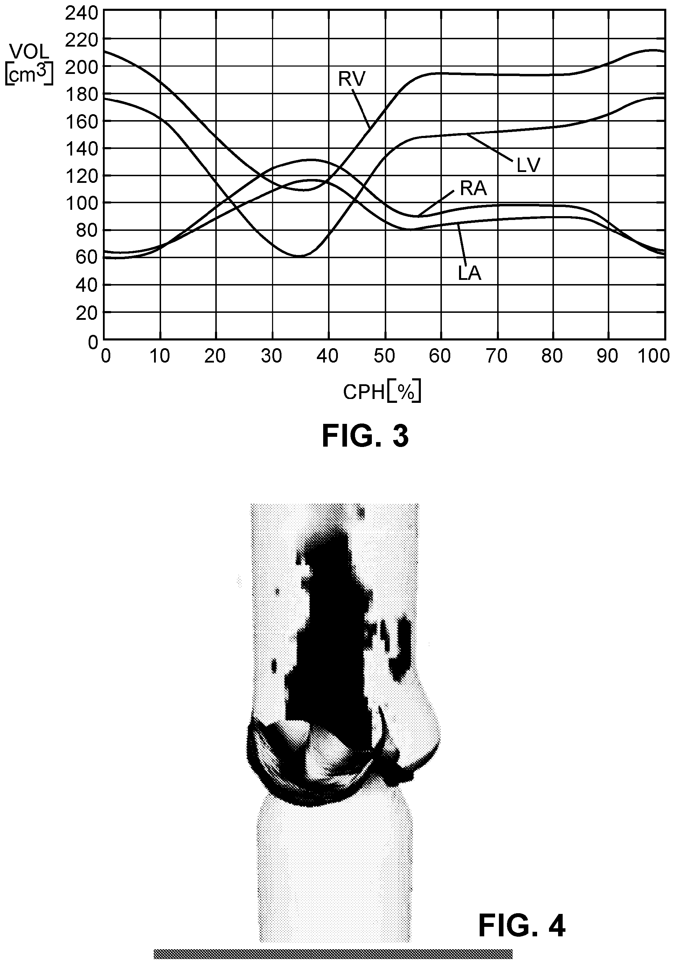

According to the present embodiment, the model-based segmentation (MBS) as described above is used in first (LVV (ED)) and second (LVV (SYS)) extracting steps or circuits 22, 23 to extract a change of the left ventricle volume (LVV) over time (cf. Fig. 3 below) from electrocardiography (ECG) gated CT images obtained in first and second imaging steps or circuits 20, 30. More specifically, the left ventricle (LV) in maximum and minimum filling state (end-diastole, end-systole) can be obtained from the first and second imaging steps 20, 30, respectively, and can be used to estimate the flow or volume or temporal behavior of the blood (i.e. cardiac ejection output) pumped per heart beat. This flow or volume or temporal behavior of the blood is then used in an estimation step or circuit (FL (AV)) 40 to define the blood flow from the left ventricular outflow tract through the aortic valve orifice into the aorta.

Figs. 2a to 2f show schematic reformatted cross-sectional top and side views, respectively, of sample results of segmented valves for open (Fig. 2a (top view), Fig. 2d (side view)), calcified medium open (Fig. 2b (top view), Fig. 2e (side view)), and closed valve (Fig. 2c (top view), Fig. 2f (side view)). The marked double arrows 100 in Figs. 2d and 2f indicate the width of the valve orifice.

Fig. 3 shows a diagram with volume curves of the four heart chambers (left atrium (LA), left ventricle (LV), right atrium (RA), right ventricle (RV)) as extracted by the model-based segmentation of the extracting steps or circuits 22, 23 from a ECG gated CT data set.

To define patient-specific boundary conditions (i.e., flow at LV outflow tract), a flow profile across the outflow tract and its temporal behavior can be estimated in the estimation step or circuit 40. For the temporal behavior, it can, for instance, be assumed that there is no flow through the aortic valve during a predetermined portion (e.g. between 40% and 10%) of the cardiac phase. In between (e.g. ∼10% - 40%), a constant flow or a volume flow curve derived from the LV volume curve in Fig. 3 may be used. The flow across the LV outflow tract can, for instance, be defined by a quadratic profile (profile for constant flow in a tube) or the velocity profile of pulsatile flow using a Womersley number.

In a subsequent CFD simulation step or circuit 50, the flow through the open valve is simulated by CFD to estimate the pressure drop for the open valve. This can be achieved by specifying the blood flow behavior and properties at the boundaries of the volumetric mesh of the blood cavity obtained from modeling step or circuit 12, while taking into account the above patient-specific boundary conditions obtained from the estimation step or circuit 40. Additionally, to obtain a complete simulation of blood flow through the aortic valve, fluid-solid interaction may be taken into account so as to also consider interactions with the elastic vascular wall. The result of the CFD simulation can be analyzed to estimate e.g. the pressure drop across the aortic valve or other physiological parameters, such as average blood residence time, flow rate, wall sheer stress and blood swirl at the aortic valve.

Finally, the results of the flow simulation can also be visualized and/or quantified in an optional visualization and/or quantification step or circuit (V/Q) 60 and virtual Doppler ultrasound images may be generated to allow a physician assessing the result. This visualization may be based on analytical methods that analyze the simulated blood flow and show properties like, e.g., streamlines, streaklines, and pathlines. The blood flow can either be given in a finite representation or as a smooth function. As an alternative, texture advection methods can be used, that "bend" textures (or images) according to the flow. Numerical values or qualitative values of one or more of the above physiological parameters may be added to the visualization to allow comparison with standard values

Fig. 4 shows a visualization of an exemplary simulation of a blood flow through an aortic valve.

The proposed solution can be used to quantify AS or other cardiovascular diseases or even other blood-flow related characteristics of cardiovascular structures by simulation in a clinical workstation or other computer system based on image data obtained from CT or MRI or ultrasound or other imaging modalities.

To summarize, a method and apparatus have been described for simulating blood flow through a patient-specific geometry of a cardiovascular structure, e.g. a blood cavity such as the left ventricle outflow tract, the aortic root including the AV, plus ascending aorta, a ventricle volume, the aorta or any other cavity where blood flows through, under patient-specific boundary conditions derived from the cardiac ejection output per heart stroke. The cardiac ejection output can be estimated from volumes of a heart chamber of the patient in different filling states at two or more different points in time. In the case of AV-related simulations, the AV geometry and the ventricle volumes can automatically be obtained by model-based segmentation. In a first step the blood cavity is segmented in a CT image. From the surface mesh, a volumetric mesh for CFD simulations can be generated. Model-based segmentation may be used to extract the volume change over time. A flow profile across the outflow tract and its temporal behavior are then defined. The result of the CFD simulation can be analyzed to estimate physiological parameters (e.g. the pressure drop etc.) across the aortic valve. The results of the flow simulation can also be visualized and "Virtual Doppler Ultrasound" images may be generated to allow a physician assessing the result.

While the invention has been illustrated and described in detail in the drawings and foregoing description, such illustration and description are to be considered illustrative or exemplary and not restrictive. The invention is not limited to the disclosed embodiment.

Other variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage.

The foregoing description details certain embodiments of the invention. It will be appreciated, however, that no matter how detailed the foregoing appears in text, the invention may be practiced in many ways, and is therefore not limited to the embodiments disclosed. It should be noted that the use of particular terminology when describing certain features or aspects of the invention should not be taken to imply that the terminology is being re-defined herein to be restricted to include any specific characteristics of the features or aspects of the invention with which that terminology is associated.

## Claims

1. An apparatus for simulating blood flow through a cardiovascular structure close to the heart of a patient, said cardiovascular structure including the left ventricular outflow tract, the aortic root including the aortic valve (AV), plus the ascending aorta, said apparatus comprising:
an estimation circuit (40) for estimating a cardiac ejection output per heart stroke based on a volume of the left ventricle (LV) of said patient in different filling states at two or more points in time, and for deriving at least one patient-specific boundary condition for the flow through said cardiovascular structure from said cardiac ejection output per heart stroke; and
a modeling circuit (12) for generating a volumetric mesh of said cardiovascular structure based on a partitioned segmented digital image of said cardiovascular structure;
a simulation circuit (50) for simulating a blood flow through said volumetric mesh of said cardiovascular structure under consideration of said patient-specific boundary conditions, to visualize said blood flow or to derive at least one physiological parameter of said patient, wherein said volumetric mesh represents said cardiovascular structure for an open valve state of said aortic valve (AV).

2. The apparatus according to claim 1, wherein said digital image is a computed tomography image or a magnetic resonance image or an ultrasonic image.

3. The apparatus according to claim 1, wherein said apparatus is adapted to derive from said simulated blood flow one or more of a pressure drop, an average blood residence time, a flow rate, a wall sheer stress and a blood swirl in said cardiovascular structure.

4. A method of simulating blood flow through a cardiovascular structure close to the heart of a patient, the cardiovascular structure including the left ventricular outflow tract, the aortic root including the aortic valve (AV), plus the ascending aorta, said method comprising:
estimating a cardiac ejection output per heart stroke based on a volume of the left ventricle (LV) of said patient in different filling states at two or more points in time;
deriving at least one patient-specific boundary condition for the flow through said cardiovascular structure from said cardiac ejection output per heart stroke; and
partitioning a digital image by using a model-based segmentation to obtain a surface mesh of said cardiovascular structure;
obtaining a volumetric mesh by converting or transforming the surface mesh into the volumetric mesh;
simulating a blood flow through said volumetric mesh of said cardiovascular structure under consideration of said patient-specific boundary conditions, to visualize said blood flow or to derive at least one physiological parameter of said patient, wherein said volumetric mesh represents said cardiovascular structure for an open valve state of said aortic valve (AV).

5. The method according to claim 4, wherein said simulation is a computational fluid dynamics simulation or a fluid-solid interaction simulation.

6. The method according to claim 4, further comprising estimating said cardiac ejection output per heart stroke based on electrocardiography gated digital images.

7. The method according to claim 4, further comprising estimating said cardiac ejection output per heart stroke based on digital images of said left ventricle (LV) in a maximum filling state and a minimum filling state.

8. The method according to claim 4, further comprising using said estimated cardiac ejection output per heart stroke to define a blood flow from said left ventricular outflow tract to said cardiovascular structure.

9. The method according to claim 8, further comprising deriving said at least one patient-specific boundary condition by estimating a flow profile across said left ventricular outflow tract and its temporal behavior.

10. The method according to claim 9, further comprising estimating said flow profile by defining a quadratic profile or a velocity profile of a pulsatile flow.

11. The method according to claim 4, further comprising estimating said cardiac ejection output per heart stroke based on volumes of said left ventricle (LV) of said patient at an end of systole and at an end of a diastole.

12. The method according to claim 4, further comprising generating virtual Doppler ultrasound images based on said simulated blood flow.

13. A computer program product comprising code means for producing the steps of claim 4 when run on a computing device.

## Patentansprüche

1. Vorrichtung zum Simulieren von Blutfluss durch eine kardiovaskuläre Struktur nahe dem Herzen eines Patienten, wobei die kardiovaskuläre Struktur den linksventrikulären Ausflusstrakt, die Aortenwurzel einschließlich der Aortenklappe (AV) und die aufsteigende Aorta beinhaltet, wobei die Vorrichtung umfasst:
eine Schätzungsschaltung (40) zum Schätzen einer kardialen Ejektionsleistung pro Schlaganfall basierend auf einem Volumen der linken Herzkammer (LV) des Patienten in verschiedenen Füllzuständen zu zwei oder mehr Zeitpunkten und zum Ableiten mindestens einer patientenspezifischen Randbedingung für den Fluss durch die kardiovaskuläre Struktur aus der kardialen Ejektionsleistung pro Schlaganfall; und
eine Modellierungsschaltung (12) zum Erzeugen eines volumetrischen Netzes der kardiovaskulären Struktur basierend auf einem partitionierten, segmentierten digitalen Bild der kardiovaskulären Struktur;
eine Simulationsschaltung (50) zum Simulieren eines Blutflusses durch das volumetrische Netz der kardiovaskulären Struktur unter Berücksichtigung der patientenspezifischen Randbedingungen, zum Visualisieren des Blutflusses oder zum Ableiten mindestens eines physiologischen Parameters des Patienten, wobei das volumetrische Netz die kardiovaskuläre Struktur für einen offenen Ventilzustand der Aortenklappe (AV) darstellt.

2. Vorrichtung nach Anspruch 1, wobei das digitale Bild ein Computertomographiebild oder ein Magnetresonanzbild oder ein Ultraschallbild ist.

3. Vorrichtung nach Anspruch 1, wobei die Vorrichtung angepasst ist, um aus dem simulierten Blutfluss einen oder mehrere eines Druckabfalls, einer durchschnittlichen Blutverweilzeit, einer Durchflussrate, einer Wand-Scherspannung und eines Blutwirbels in der kardiovaskulären Struktur abzuleiten.

4. Verfahren zum Simulieren von Blutfluss durch eine kardiovaskuläre Struktur nahe dem Herzen eines Patienten, wobei die kardiovaskuläre Struktur den linksventrikulären Ausflusstrakt, die Aortenwurzel einschließlich der Aortenklappe (AV) und die aufsteigende Aorta beinhaltet, wobei das Verfahren umfasst:
Schätzen einer kardialen Ejektionsleistung pro Schlaganfall basierend auf einem Volumen der linken Herzkammer (LV) des Patienten in verschiedenen Füllzuständen zu zwei oder mehr Zeitpunkten;
Ableiten mindestens einer patientenspezifischen Randbedingung für den Fluss durch die kardiovaskuläre Struktur aus dem kardialen Ejektionsleistung pro Schlaganfall; und
Partitionieren eines digitalen Bildes unter Verwendung einer modellbasierten Segmentierung, um ein Oberflächennetz der kardiovaskulären Struktur zu erhalten;
Erhalten eines volumetrischen Netzes durch Konvertieren oder Umwandeln des Oberflächennetzes in das volumetrische Netz;
Simulieren eines Blutflusses durch das volumetrische Netz der kardiovaskulären Struktur unter Berücksichtigung der patientenspezifischen Randbedingungen, zum Visualisieren des Blutflusses oder zum Ableiten mindestens eines physiologischen Parameters des Patienten, wobei das volumetrische Netz die kardiovaskuläre Struktur für einen offenen Ventilzustand der Aortenklappe (AV) darstellt.

5. Verfahren nach Anspruch 4, wobei die Simulation eine rechnerische Fluiddynamik-Simulation oder eine Fluid-Feststoff-Interaktionssimulation ist.

6. Verfahren nach Anspruch 4, weiter umfassend das Schätzen der kardialen Ejektionsleistung pro Schlaganfall basierend auf elektrokardiographisch gattergesteuerten digitalen Bildern.

7. Verfahren nach Anspruch 4, weiter umfassend das Schätzen der kardialen Ejektionsleistung pro Schlaganfall basierend auf digitalen Bildern der linken Herzkammer (LV) in einem maximalen Füllzustand und einem minimalen Füllzustand.

8. Verfahren nach Anspruch 4, weiter umfassend die Verwendung des geschätzten kardialen Ejektionsleistung pro Schlaganfall, um einen Blutfluss vom linksventrikulären Ausflusstrakt zur kardiovaskulären Struktur zu definieren.

9. Verfahren nach Anspruch 8, weiter umfassend das Ableiten der mindestens einen patientenspezifischen Randbedingung durch Schätzen eines Flussprofils über den linksventrikulären Ausflusstrakt und dessen zeitliches Verhalten.

10. Verfahren nach Anspruch 9, weiter umfassend das Schätzen des Flussprofils durch Definieren eines quadratischen Profils oder eines Geschwindigkeitsprofils eines pulsierenden Flusses.

11. Verfahren nach Anspruch 4, weiter umfassend das Schätzen der kardialen Ejektionsleistung pro Schlaganfall basierend auf Volumina der linken Herzkammer (LV) des Patienten an einem Ende der Systole und an einem Ende einer Diastole.

12. Verfahren nach Anspruch 4, weiter umfassend das Erzeugen virtueller Doppler-Ultraschallbilder basierend auf dem simulierten Blutfluss.

13. Computerprogrammprodukt, umfassend Codemittel zum Erzeugen der Schritte nach Anspruch 4, wenn es auf einer Computervorrichtung ausgeführt wird.

## Revendications

1. Appareil pour simuler un flux sanguin à travers une structure cardiovasculaire proche du coeur d'un patient, ladite structure cardiovasculaire incluant la voie de sortie ventriculaire gauche, la racine aortique incluant la valve aortique (AV), plus l'aorte ascendante, ledit appareil comprenant :
un circuit d'estimation (40) pour estimer une sortie d'éjection cardiaque par battement de coeur sur la base d'un volume du ventricule gauche (LV) dudit patient dans différents états de remplissage à deux ou plus de deux instants, et pour dériver au moins une condition limite spécifique au patient pour le flux à travers ladite structure cardiovasculaire à partir de ladite sortie d'éjection cardiaque par battement de coeur ; et
un circuit de modélisation (12) pour générer une maille volumétrique de ladite structure cardiovasculaire sur la base d'une image numérique segmentée partitionnée de ladite structure cardiovasculaire ;
un circuit de simulation (50) pour simuler un flux sanguin à travers ladite maille volumétrique de ladite structure cardiovasculaire en tenant compte desdites conditions limites spécifiques au patient, pour visualiser ledit flux sanguin ou pour dériver au moins un paramètre physiologique dudit patient, ladite maille volumétrique représentant ladite structure cardiovasculaire pour un état valvulaire ouvert de ladite valve aortique (AV).

2. Appareil selon la revendication 1, dans lequel ladite image numérique est une image de tomographie assistée par ordinateur ou une image de résonance magnétique ou une image ultrasonore.

3. Appareil selon la revendication 1, dans lequel ledit appareil est adapté pour dériver dudit flux sanguin simulé une ou plusieurs valeurs parmi une chute de pression, un temps de séjour sanguin moyen, un débit, une contrainte de cisaillement de paroi et un tourbillon sanguin dans ladite structure cardiovasculaire.

4. Procédé de simulation de flux sanguin à travers une structure cardiovasculaire proche du coeur d'un patient, la structure cardiovasculaire incluant la voie de sortie ventriculaire gauche, la racine aortique incluant la valve aortique (AV), plus l'aorte ascendante, ledit procédé comprenant les étapes consistant à :
estimer une sortie d'éjection cardiaque par battement de coeur sur la base d'un volume du ventricule gauche (LV) dudit patient dans différents états de remplissage à deux ou plus de deux instants ;
dériver au moins une condition limite spécifique au patient pour le flux à travers ladite structure cardiovasculaire à partir de ladite sortie d'éjection cardiaque par battement de coeur ; et
partitionner une image numérique en utilisant une segmentation basée sur un modèle pour obtenir une maille de surface de ladite structure cardiovasculaire ;
obtenir une maille volumétrique en convertissant ou en transformant la maille de surface en la maille volumétrique ;
simuler un flux sanguin à travers ladite maille volumétrique de ladite structure cardiovasculaire en tenant compte desdites conditions limites spécifiques au patient, pour visualiser ledit flux sanguin ou dériver au moins un paramètre physiologique dudit patient, ladite maille volumétrique représentant ladite structure cardiovasculaire pour un état valvulaire ouvert de ladite valve aortique (AV).

5. Procédé selon la revendication 4, dans lequel ladite simulation est une simulation numérique de dynamique des fluides ou une simulation d'interaction fluide-solide.

6. Procédé selon la revendication 4, comprenant en outre l'estimation de ladite sortie d'éjection cardiaque par battement de coeur sur la base d'images numériques déclenchées par électrocardiographie.

7. Procédé selon la revendication 4, comprenant en outre l'estimation de ladite sortie d'éjection cardiaque par battement de coeur sur la base d'images numériques dudit ventricule gauche (LV) dans un état de remplissage maximal et dans un état de remplissage minimal.

8. Procédé selon la revendication 4, comprenant en outre l'utilisation de ladite sortie d'éjection cardiaque estimée par battement de coeur pour définir un flux sanguin depuis ladite voie de sortie ventriculaire gauche vers ladite structure cardiovasculaire.

9. Procédé selon la revendication 8, comprenant en outre la dérivation de ladite au moins une condition limite spécifique au patient en estimant un profil de flux à travers ladite voie de sortie ventriculaire gauche et son comportement temporel.

10. Procédé selon la revendication 9, comprenant en outre l'estimation dudit profil de flux en définissant un profil quadratique ou un profil de vitesse d'un flux pulsatile.

11. Procédé selon la revendication 4, comprenant en outre l'estimation de ladite sortie d'éjection cardiaque par battement de coeur sur la base de volumes dudit ventricule gauche (LV) dudit patient à une fin de systole et à une fin de diastole.

12. Procédé selon la revendication 4, comprenant en outre la génération d'images ultrasonores Doppler virtuelles basées sur ledit flux sanguin simulé.

13. Produit de programme d'ordinateur comprenant des moyens de code pour produire les étapes selon la revendication 4 lorsqu'il est exécuté sur un dispositif informatique.
